(19) Europäisches Patentamt
European Patent Office
Office européen des brevets

(11) **EP 4 129 159 B1**

(12) **EUROPEAN PATENT SPECIFICATION**

(45) Date of publication and mention
of the grant of the patent:
**18.02.2026 Bulletin 2026/08**

(21) Application number: **21793413.2**

(22) Date of filing: **15.04.2021**

(51) International Patent Classification (IPC):
*A61B 5/00* (2006.01)      *A61B 5/01* (2006.01)

(52) Cooperative Patent Classification (CPC):
**A61B 5/0008; A61B 5/7225;** A61B 5/0006;
A61B 5/0024; A61B 5/02125; Y02A 90/10

(86) International application number:
**PCT/JP2021/015578**

(87) International publication number:
**WO 2021/215342 (28.10.2021 Gazette 2021/43)**

(54) **CONVERSION ADAPTER**

WANDLERADAPTER

ADAPTATEUR DE CONVERSION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **21.04.2020 JP 2020075300**

(43) Date of publication of application:
**08.02.2023 Bulletin 2023/06**

(73) Proprietor: **MURATA Manufacturing Co., Ltd.
Nagaokakyo-shi, Kyoto 617-8555 (JP)**

(72) Inventors:
• **ICHIKAWA, Hiroaki
Nagaokakyo-shi, Kyoto 617-8555 (JP)**
• **UEGAKI, Hidesato
Nagaokakyo-shi, Kyoto 617-8555 (JP)**
• **SHIBATA, Akihiko
Nagaokakyo-shi, Kyoto 617-8555 (JP)**
• **EJIRI, Kouki
Nagaokakyo-shi, Kyoto 617-8555 (JP)**
• **ASAI, Kiyotaka
Nagaokakyo-shi, Kyoto 617-8555 (JP)**

• **TSUCHIMOTO, Hirofumi
Nagaokakyo-shi, Kyoto 617-8555 (JP)**
• **TANAKA, Koji
Nagaokakyo-shi, Kyoto 617-8555 (JP)**
• **TSUBAKIZAKA, Kota
Nagaokakyo-shi, Kyoto 617-8555 (JP)**
• **KIMURA, Shuto
Nagaokakyo-shi, Kyoto 617-8555 (JP)**

(74) Representative: **Lorenz Seidler Gossel Part. mbB
Widenmayerstr. 23
80538 München (DE)**

(56) References cited:
WO-A1-2011/129418      WO-A1-2017/170212
JP-A- 2009 183 721      JP-A- 2013 526 918
JP-A- 2014 532 543      JP-A- 2016 057 961
JP-A- 2017 131 319      JP-A- 2018 152 754
US-A1- 2003 040 305      US-A1- 2004 111 043
US-A1- 2005 119 533      US-A1- 2012 123 692
US-A1- 2014 091 947      US-A1- 2016 317 055
US-A1- 2020 060 590

EP 4 129 159 B1

**Description**

Technical Field

The present invention relates to a conversion adapter.

Background Art

To date, a biological information monitor (bedside monitor) has been used in the hospital (sickroom) and the like to monitor the condition of a patient. The patient wears a sensor, and the biological information monitor thus monitors, for example, biological information (vital signs) such as blood pressure, body temperature, respiration, and a pulse rate of the patient.

Patent Document 1 discloses a wired biological information monitor including a biological sensor connected to the biological information monitor (bedside monitor) with cables (through wire). More specifically, the biological information monitor includes a display unit and a plurality of connectors provided in the lower part of the display unit, and the cables with sensors are connected to the plurality of respective connectors to monitor biological information.

Patent Document 2 discloses a wireless biological information monitor that includes a wireless sensor for a patient (biological sensor) and to which the wireless sensor is connected wirelessly, the wireless sensor wirelessly transmitting biological information to the biological information monitor.

Citation List

Patent Documents

Patent Document 1: Japanese Unexamined Patent Application Publication No. 2007-215582
Patent Document 2: Japanese Unexamined Patent Application Publication (Translation of PCT Application) No. 2018-527996
US 2005/119533 A1 discloses a radiofrequency adapter for medical monitoring equipment, the adapter permitting the use of known types of sensor and known types of monitor, but without requiring the use of connection cables between the sensor and the monitor.

Another prior art system comprising a conversion adapter is known from US 2005/119533.

Summary of Invention

Technical Problem

In the biological information monitor described in Patent Document 1 above, the biological sensor worn by a patient and the biological information monitor are connected by using the cables (through wire), and thus there is a problem such as restrained movement of the patient due to the cables.

In contrast, with the wireless biological information monitor described in Patent Document 2, the above-described problem can be solved. However, for example, if a hospital or the like using a wired biological information monitor buys a relatively expensive wireless biological information monitor to replace the wired biological information monitor, the buying leads to a high burden from a viewpoint of cost.

There has thus been a desire that a wireless capability be added without replacing an existing device, that is, the wired biological-information processing apparatus (such as the biological information monitor).

The present invention has been made to solve the aforementioned problem, and it is an object to provide a conversion adapter that enables a biological-information processing apparatus (such as a biological information monitor) with wired connection to a biological sensor or another device to be used simply and at low cost as a wireless biological-information processing apparatus (that is, the wireless capability to be added).

Solution to Problem

A conversion adapter according to the present invention is defined in claim 1.

According to the conversion adapter of the present invention, the biological information based on the digital data transmitted from the external apparatus is received by using radio communication. After being subjected to data processing and converted to the biological information conforming to the external biological-information processing apparatus, the received biological information is output by using the connection unit.

Advantageous Effects of Invention

As the result, according to the present invention, a biological-information processing apparatus (such as a biological information monitor) connected to the biological sensor or another device through wire can be used simply and at low cost as a wireless biological-information processing apparatus (that is, the wireless capability can be added). Brief Description of Drawings

[Fig. 1] Fig. 1 is a view illustrating respective examples of a conversion adapter according to an embodiment and a biological information monitor (bedside monitor) to which the conversion adapter is applied.
[Fig. 2] Fig. 2 is a view illustrating the external appearance of the conversion adapter according to the embodiment.
[Fig. 3] Fig. 3 is a block diagram illustrating the functional configuration of the conversion adapter according to the embodiment.
[Fig. 4] Fig. 4 illustrates graphs of temperature output characteristics before calibration and temperature output characteristics after the calibration.
[Fig. 5] Fig. 5 is a view for explaining a deep-body temperature estimation process.
[Fig. 6] Fig. 6 is a graph illustrating an example of a photoplethysmogram.
[Fig. 7] Fig. 7 is a view for explaining a method for calculating a blood sugar level (blood pressure variation).
[Fig. 8] Fig. 8 is a diagram illustrating the configuration of a converter of the conversion adapter according to the embodiment.
[Fig. 9] Fig. 9 is a graph illustrating switching resistance distribution of a plurality of switching elements constituting the converter (total switch resistance value distribution graph).
[Fig. 10] Fig. 10 is a block diagram illustrating the configuration of a conversion adapter according to a first modification.
[Fig. 11] Fig. 11 is a block diagram illustrating the configuration of a conversion adapter according to a second modification.
[Fig. 12] Fig. 12 is a view for explaining a connection verification (pairing verification) method.
[Fig. 13] Fig. 13 is a block diagram illustrating the configuration of a conversion adapter according to a third modification.
[Fig. 14] Fig. 14 is a flowchart illustrating the processing steps of a fault diagnosis process performed by the converter of the conversion adapter according to the embodiment.

Description of Embodiments

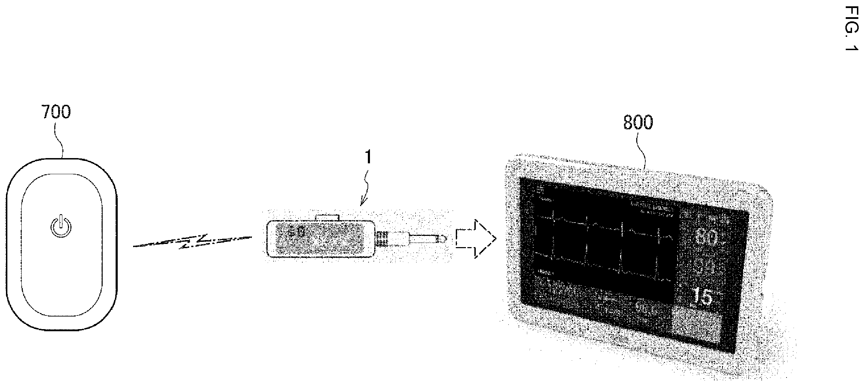

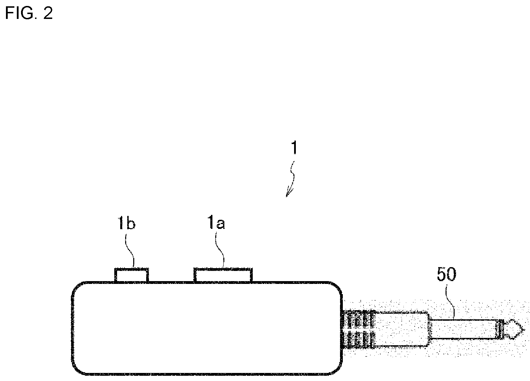

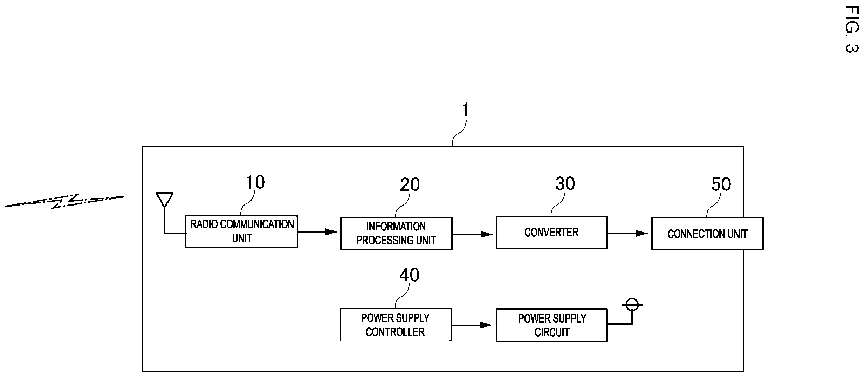

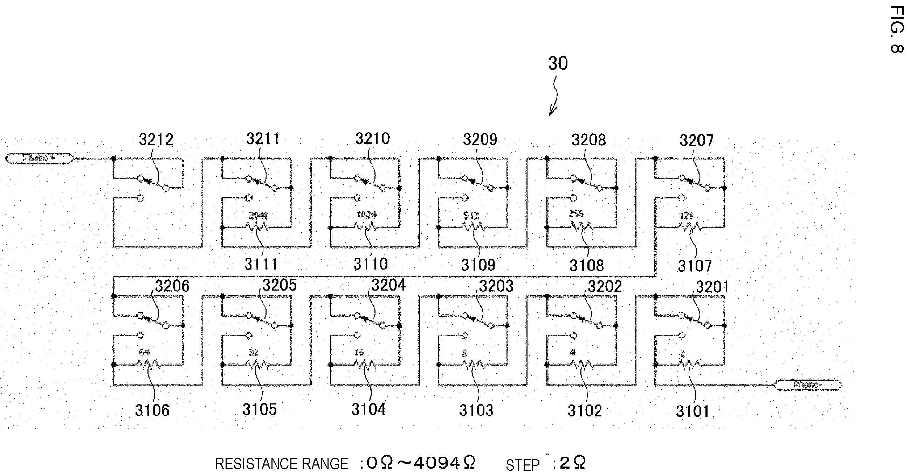

Hereinafter, a preferable embodiment of the present invention will be described in detail with reference to the drawings. Note that the same reference numerals are used as those for portions or corresponding portions throughout the drawings. The same components are denoted by the same reference numerals throughout the drawings, and repeated description thereof is omitted.
First, the configuration of a conversion adapter 1 according to the embodiment will be described by using Figs. 1 to 9. Fig. 1 is a view illustrating respective examples of the conversion adapter 1 and a biological information monitor 800 to which the conversion adapter 1 is applied. Fig. 2 is a view illustrating the external appearance of the conversion adapter 1. Fig. 3 is a block diagram of the functional configuration of the conversion adapter 1. Fig. 8 is a block diagram illustrating the configuration of a converter 30 of the conversion adapter 1.
The conversion adapter 1 is a conversion device used to connect a wireless biological sensor 700 to the wired biological information monitor (a bedside monitor) 800. The conversion adapter 1 enables the connection therebetween by absorbing a difference in standard, voltage, and the like between the wireless biological sensor 700 and the wired biological information monitor 800. Note that the details will be described later.
The biological sensor 700 is an attached biological sensor that is worn by a measurement target (for example, a patient in hospital) and that detects biological information from the measurement target. As the biological information, for example, body temperature, blood pressure, a blood sugar level, oxygen saturation, a heart rate, ECG, and PPG can be cited. Any of various biological sensors (such as a body temperature sensor, a photoplethysmographic sensor, a pressure sensor, a blood sugar level sensor, an oxygen saturation sensor, a pulse wave sensor, and an electrocardiographic electrode) that detect (acquire) these pieces of biological information is thus used as the biological sensor 700. In this embodiment, a case where a temperature sensor and a photoplethysmographic sensor are used as the biological sensor 700 will be described as an example. The temperature sensor uses a thermistor having a resistance value that varies depending on the temperature, and the photoplethysmographic sensor has a light emitting element and a light receiving element and optically detects a photoplethysmographic signal by using the absorption characteristics of blood hemoglobin.

The biological sensor 700 includes an A/D converter that converts an analog signal (biological signal) to digital data. The biological sensor 700 converts a detected biological signal to digital data (biological information). The biological sensor 700 also includes a radio communication module and wirelessly transmits the digital data (biological information) subjected to the A/D conversion. Note that the digital data (biological information) may be a physical quantity such as temperature or a pressure and may also be waveform data such as an electrocardiogram. For the radio communication, radio communication methods (radio communication standards) such as Bluetooth (registered trademark) low energy (BLE), WiFi, long term evolution (LTE), and Sub-GHz (a 900 MHz band) are used. Radio communication methods (radio communication standards) such as near field communication (NFC) (ISO/IEC 18092) and MIFARE (registered trademark) (ISO/IEC 14443) may also be used.

For the biological information monitor (corresponding to a biological-information processing apparatus described in the scope of claims) 800, a publicly known wired and analog-input biological information monitor can be used. The biological information monitor 800 includes a display screen and receives and displays the biological information that is detected, converted, and transmitted by the biological sensor 700 and that is received, converted, and relayed via the conversion adapter 1 (described later).

As described above, the conversion adapter 1 is the conversion device used to wirelessly connect the wireless biological sensor 700 to the wired biological information monitor 800. The conversion adapter 1 enables the connection therebetween by absorbing a difference in standard, voltage, and the like between the wireless biological sensor 700 and the wired biological information monitor 800. In particular, the conversion adapter 1 has a function of enabling the wired biological information monitor 800 to be used simply and at low cost as a wireless biological information monitor (that is, a wireless capability to be added).

Accordingly, the conversion adapter 1 mainly includes a radio communication unit 10, an information processing unit 20, the converter 30, a power supply controller 40, and a connection unit 50.

The radio communication unit 10 receives, from the external biological sensor 700, the biological information based on the digital data transmitted by using radio communication. For example, the radio communication unit 10 receives a plurality of pieces of temperature information, photoplethysmographic information, and the like as the biological information. For the radio communication unit 10, a radio communication method (radio communication standard) such as Bluetooth (registered trademark) low energy (BLE), WiFi, long term evolution (LTE), or Sub-GHz (a 900 MHz band) is used to suit the radio communication method for the biological sensor 700 described above. A radio communication method (radio communication standard) such as near field communication (NFC) (ISO/IEC 18092) or MIFARE (registered trademark) (ISO/IEC 14443) may also be used. Note that the biological information received by the radio communication unit 10 is output to the information processing unit 20.

The radio communication unit 10 selects, for example, one of biological sensors 700 that has the highest radio wave intensity of the received radio wave as the biological sensor 700 with which connection (pairing) is to be established. That is, if the plurality of biological sensors 700 are detected, the radio communication unit 10 performs connection to the biological sensor 700 having the highest radio wave intensity. In this case, there is not a connection user interface (UI) between the conversion adapter 1 and the biological sensor 700, and the connection is performed automatically. Note that if the radio wave intensity of the received radio wave (the detected biological sensor 700) varies by a predetermined width or more, the radio communication unit 10 preferably outputs information indicating that the radio wave intensity varies by the predetermined width or more to report to a user by displaying, sound, or the like. Likewise, if the radio wave intensity has dropped to a predetermined value or lower (has become lower than a reception sensitivity point) before or after the establishment of the pairing, the radio communication unit 10 preferably outputs information indicating that the radio wave intensity has dropped to the predetermined value or lower (has become lower than the reception sensitivity point) to report to the information by displaing, sound, or the like.

Note that instead of the above-described connection method, a configuration in which pressing a connection switch 1b for establishing the connection with the biological sensor 700 causes the connection (pairing) with the biological sensor 700 may be employed.

The conversion adapter 1 also includes a memory that stores the biological sensor 700 with which the connection is established. If the biological sensor 700 is disconnected, the radio communication unit 10 preferably retries the connection with the biological sensor 700 stored in the memory. This is performed to prevent wrong connection after the disconnection.

The information processing unit 20 includes a micro control unit (MCU), a memory (corresponding to a memory described in the scope of claims), or the like. The information processing unit 20 performs data processing of the biological information based on the digital data received by the radio communication unit 10 and generates significant data to be displayed and used by the biological information monitor 800.

The information processing unit 20 particularly performs an algorithmic process on the biological information based on the digital data. In this embodiment, in the algorithmic process, for example, a calibration process for correcting individual variation of the biological sensor 700, a deep-body temperature estimation process for estimating deep-body temperature based on a plurality of pieces of temperature information, a balance determination process for determining whether the biological sensor 700 (temperature sensor) is thermally balanced (in a state without heat flow rate variation), and a process for estimating a blood pressure level or the like in which a blood pressure level and a blood sugar level are estimated based on photoplethysmographic information are performed. The processes will herein be described.

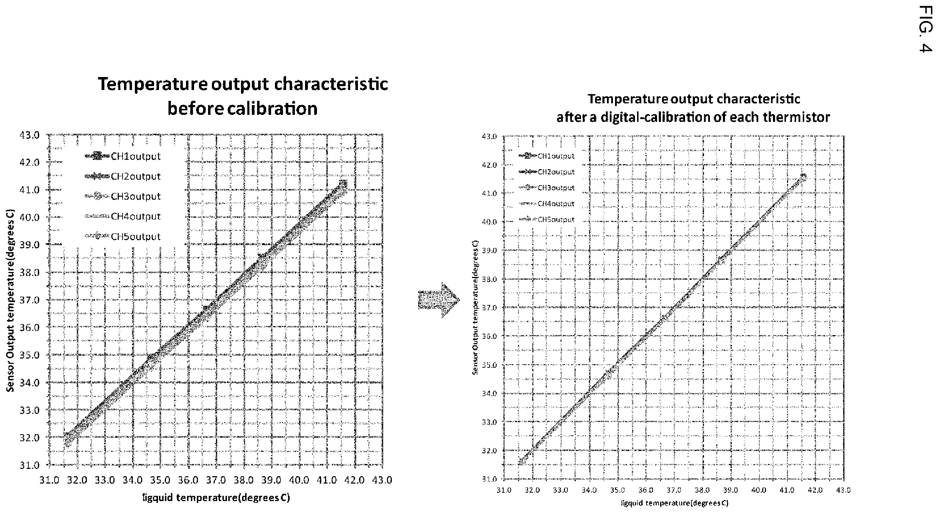

First, as illustrated in Fig. 4, the information processing unit 20 converts a digital value x to temperature data Y by using the fitting curve "$Y = ax^2 + bx + c$". At this time, the voltage output for each temperature is output as an offset difference due to the influence of characteristic variation (individual variation) and the like in thermisters. The information processing unit 20 thus performs a calibration process for correcting the characteristic variation (individual variation) of the biological sensor 700. More specifically, as illustrated in Fig. 4, the c constant of the fitting curve is calibrated for each individual, and the influence of the variation due to an individual difference is thereby reduced. Note that Fig. 4 illustrates graphs of the temperature output characteristics before the calibration (left graph) and the temperature output characteristics after the calibration (right graph). The horizontal axis in Fig. 4 represents temperature (°C), and the vertical axis represents value (digital value) resulting from the digital conversion of output voltages (partial pressure values) of the biological sensor 700 (thermisters). In the calibration, a polynomial is calculated after temperature characteristics (temperature - digital output) are measured in a state where digital output value variation in UART output is reduced due to power supply from a DC power supply, and the c constant is obtained such that a digital value at 37°C matches the actual measured temperature. At this time, the c constant is obtained by using the mean value of inclinations a, b of, for example, five (CH1 to CH5) temperature sensors. The curve fitting and the offset value per individual (c constant) that are thus obtained are stored as the calibration information in the EEPRROM or the like, and temperature output with a small deviation ($\pm 0.05$°C) from the reference (platinum thermometer) is obtained (see the right graph in Fig. 4).

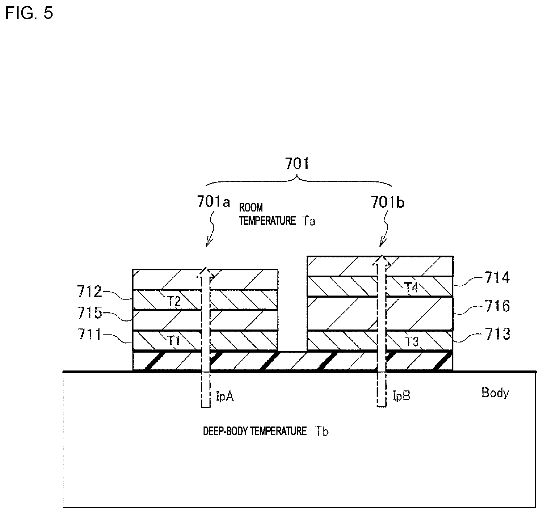

The information processing unit 20 also estimates deep-body temperature based on the plurality of pieces of temperature information. Fig. 5 is a view for explaining a deep-body temperature estimation process. The information processing unit 20 obtains body temperature data (deep-body temperature) based on a thermal resistance value RpA of a thermal resistance element 715 that is stored in advance, a detected temperature T1 of a first temperature sensor 711, a detected temperature T2 of a second temperature sensor 712, a thermal resistance value RpB of a thermal resistance element 716 that is stored in advance, a detected temperature T3 of a third temperature sensor 713, and a detected temperature T4 of a fourth temperature sensor 714.

More specifically, by using the following formulas (1) and (2), the information processing unit 20 compares heat flows in two systems having different thermal resistance elements (thermal resistances), erases an unknown thermal resistance RB, and calculates (estimates) a body-temperature data candidate Tb of a user (human body) having the unknown thermal resistance RB.

$$\text{Heat flow IpA} = (T1-T2)/RpA = (Tb-T1)/RB \text{ ... (1)}$$

$$\text{Heat flow IpB} = (T3-T4)/RpB = (Tb-T3)/RB \text{ ... (2)}$$

RpA and RpB denote the thermal resistances (known) of the thermal resistance elements 715 and 716.

If the thermal resistance RB of the user (human body) is known, a body-temperature data candidate can be calculated (estimated) by using one of sensing parts, that is, a sensing part 701a (or 701b). In more detail, in a case where the body-temperature data candidate of the human body is Tb, the detected temperature of the first temperature sensor 711 is T1, the detected temperature of the second temperature sensor 712 is T2, an equivalent thermal resistance from the deep part of the human body to the surface is RB, and an equivalent thermal resistance of the thickness direction of the thermal resistance element 715 is RpA, the body-temperature data candidate Tb that reaches a thermally balanced state can be expressed as in the following formula (3).

$$Tb = T2 + \{RpA/(RB + RpA)\}(T1-T2) \text{ ... (3)}$$

Thus, in the case where the thermal resistance RB of the human body is known, or by setting, for example, a typical (standard) thermal resistance value as the thermal resistance RB of the human body, a deep-body temperature Tb can be obtained from the temperature T1 detected by the first temperature sensor 711 and the temperature T2 detected by the second temperature sensor 712.

When obtaining the deep-body temperature Tb, the information processing unit 20 acquires the body temperature data based on temperature data detected when it is determined that the biological sensor 700 is thermally balanced. The information processing unit 20 determines whether the biological sensor 700 (temperature sensors) is thermally balanced (in a state without heat flow rate variation) by using a balanced-state discrimination formula. More specifically, the information processing unit 20 determines whether the biological sensor 700 (temperature sensors) is thermally balanced by using the following balanced-state discrimination formula (4). That is, in a case where the temperature data detected by the first temperature sensor 711 is T1, the temperature data detected by the second temperature sensor 712 is T2, the temperature data detected by the third temperature sensor 713 is T3, the temperature data detected by the fourth temperature sensor 714 is T4, and if the balanced-state discrimination formula (4) is satisfied, the information processing unit 20 determines that the biological sensor 700 is thermally balanced. In contrast, if the balanced-state discrimination formula (4) is not satisfied, the information processing unit 20 determines that the biological sensor 700 is not thermally balanced (not balanced).

$$T3-T4 > T1-T2, \quad T3 > T1 \ \dots \ (4)$$

Note that if an room temperature Ta in the hospital can be acquired from, for example, an electric medical record system (or an infection management system), whether a temperature detection part 701 (the four temperature sensors 711 to 714) is thermally balanced may be determined further in consideration for the balanced-state discrimination formulas (5), (6), and (7). In this case, if the balanced-state discrimination formulas (5), (6), and (7) in addition to the balanced-state discrimination formula (4) above are all satisfied, it is determined that the biological sensor 700 is thermally balanced. In contrast, if any one or all of the balanced-state discrimination formulas (4) to (7) are not satisfied, it is determined that the biological sensor 700 is not thermally balanced (not balanced).

$$dTa > dT4 \ \dots \ (5)$$

$$K(T1-T2) - (T3-T4) > 0 \ (\text{when } Ta > Tb) \ \dots \ (6)$$

$$K(T1-T2) - (T3-T4) \leq 0 \ (\text{when } Ta \leq Tb) \ \dots \ (7)$$

A constant K is a ratio of the thermal resistances in the two heat flows.

The information processing unit 20 also estimates a blood pressure level based on the photoplethysmographic information. In more detail, the information processing unit 20 estimates blood pressure (an absolute value) from the following formula (8).

Blood pressure (absolute value) = normal blood- pressure level + blood pressure variation          (8)

The blood pressure variation is obtained from the following formula.

$$\text{Blood pressure variation} = A \cdot \Delta \text{ pulse rate} + b$$

$$= c \cdot \Delta RI + d$$

$$= e \cdot \Delta T + f$$

Note that a, b, c, d, e, and f are coefficients (fixed values) specific to the pulse wave device.

The $\Delta$ pulse rate is an increase or decrease value of a pulse rate (the number of times a pulse wave waves in a minute).

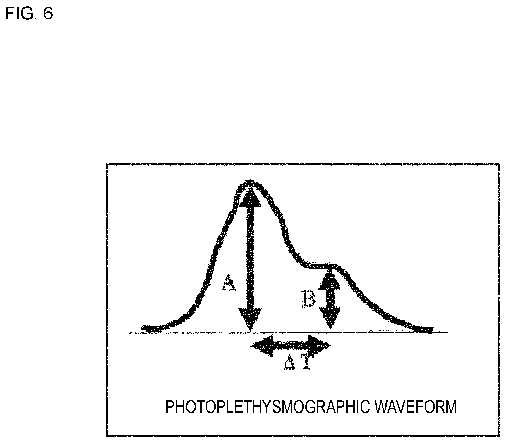

$\Delta RI$ (reflection index) is an increase or decrease value of the RI. The RI is the intensity of the pulse wave reflection in the periphery and is obtained from a ratio between an ejection wave (amplitude A) and a reflected wave (amplitude B) in the photoplethysmogram (see Fig. 6). Fig. 6 is a graph illustrating an example of the photoplethysmogram.

$\Delta(\Delta T)$ is an increase or decrease value of $\Delta T$ (a period of time between the ejection wave and the reflected wave (see Fig. 6)).

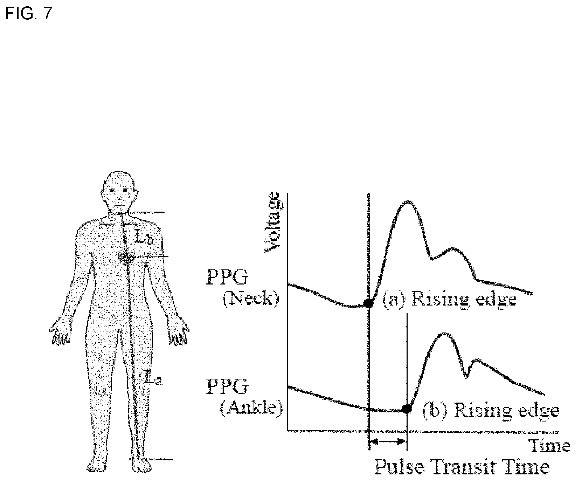

Further, the information processing unit 20 estimates the blood sugar level based on the photoplethysmographic information. Fig. 7 is a view for explaining a method for calculating a blood sugar level (blood-sugar level variation). In more detail, the information processing unit 20 estimates the blood sugar level (absolute value) from the following formula (9).

Blood sugar (absolute value) = normal blood-sugar level + blood-sugar level variation

The blood-sugar level variation is obtained from the following formula.

$$\text{Blood-sugar level variation} = g \cdot \Delta \text{PTT} + h$$

Note that g and h denote coefficients (fixed values) specific to the pulse wave device and the measurement portion.

Thereafter (that is, after the processes described above), the information processing unit 20 converts the biological information (data) obtained in the processes described above to a digital value corresponding to an analog amount for the biological information monitor 800. The conversion can be performed based on, for example, a transformation or a reference table. The biological information based on the digital data processed by the information processing unit 20 is output to the converter 30.

The converter 30 converts the biological information based on the digital data processed by the information processing unit 20 to the biological information (signal) based on analog data. For example, the converter 30 converts the biological information (digital value) to a resistance value corresponding to the resistance value (analog data) of the thermisters (biological sensor 700). Note that the analog amount is not limited to the resistance value and may be, for example, a current value or a voltage value.

The case of the conversion to the resistance value will herein be described as an example. In this case, as illustrated in Fig. 8, the converter 30 includes a plurality of resistors 3101 to 3111 connected in series (11 resistors in the example illustrated in Fig. 8) and switching elements 3201 to 3212 that are respectively connected in parallel to the plurality of resistors 3101 to 3111 and that respectively turn on (enable) and off (disable) the plurality of resistors 3101 to 3111. The respective resistance values of the plurality of (11) resistors 3101 to 3111 are each set at $2^1$ to $2^n \, \Omega$ (n is a natural number). The respective resistance values of the plurality of (11) resistors 3101 to 3111 are thus respectively set at 2, 4, 8, 16, 32, 64, 128, 256, 512, 1024, and 2048 ($\Omega$). The resistors 3101 to 3111 may each be composed of one resistor or a plurality of resistors connected in series or in parallel.

The converter 30 obtains a resistance value (analog value) of thermisters corresponding to the biological information (digital value) and obtains a combination of the plurality of (11) resistors 3101 to 3111 such that the resistance value of the thermisters corresponding to the obtained biological information matches a combined series resistance value, decides a combination of the turning on and off of the switching elements 3201 to 3212 to achieve the obtained combination of the resistors 3101 to 3111, and turns on or off the switching elements 3201 to 3212. The processing and the control are performed by the MCU. If the resistance value is controlled by the 12 switching elements 3201 to 3212, a deep-body temperature of 37.0°C leads to, for example, the combination "switching element 3201: ON, switching element 3202: OFF, ..., switching element 3212: ON", and a deep-body temperature of 37.5°C leads to, for example, the combination "switching element 3201: OFF, switching element 3202: ON, ..., switching element 3212: ON".

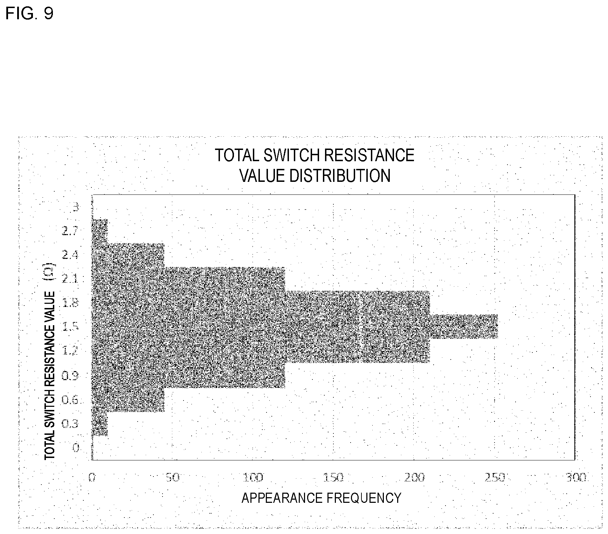

At this time, the converter 30 preferably corrects (performs subtraction of) the resistance value by using the median (1.5 $\Omega$ in the example in Fig. 9) of the switching resistance of the switching elements 3201 to 3212 as a correction value, as illustrated in Fig. 9. The resistance value (combined series resistance value) converted by the converter 30 is output to the connection unit 50.

The converter 30 outputs the resistance value corresponding to the temperature data (biological information) after combining the resistance values of $2^1$ to $2^n \, \Omega$, but there is a risk that variation in an on resistance among the resistors 3101 to 3111 and the switching elements 3201 to 3212 prevents an ideal resistance value from being output. The information processing unit 20 thus performs a calibration process (resistance calibration) for correcting the variation of resistance values of the resistors 3101 to 3111. More specifically, the information processing unit 20 measures an output resistance value for any input temperature data (by using a measurement device inside or outside the convertion adapter 1), compares an ideal output resistance value for the input temperature data with the measured resistance value, stores a difference therebetween in the MCU (memory), and performs offset correction.

Further, the information processing unit 20 performs a fault diagnosis (self-check) for the converter 30 including the plurality of resistors 3101 to 3111. The information processing unit 20 thus checks whther the resistance value to be output to the biological information monitor 800 is appropriate. If there is an anormaly, the information processing unit 20 reports to the user. More specifically, when the conversion adapter 1 is powered on, the information processing unit 20 applies a predetermined voltage betwen Phone+ and Phone- (see Fig. 8). The information processing unit 20 reads a voltage value (patial voltage) of $2^1$ to $2^n \, \Omega$ applied to each of the resistors 3101 to 3111. If the voltage value deviates from a range set in advance (reference value), the information processing unit 20 determines the conversion adapter 1 as having a fault.

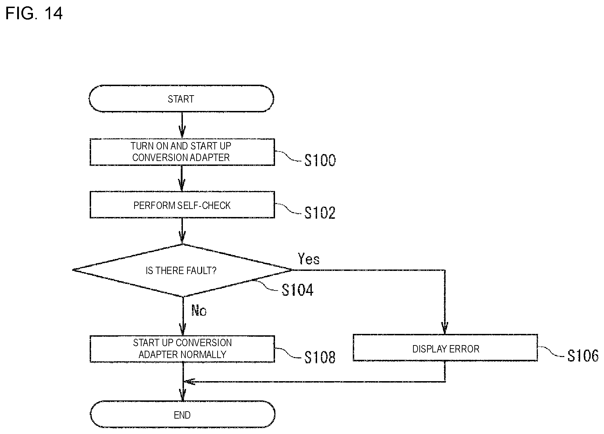

Fig. 14 is a flowchart of processing steps in the fault diagnosis process performed for the converter 30 by the conversion adapter 1. After the conversion adapter 1 is powered on and started (step S100), an operation for the self-check function is automatically started, and the self-check is performed (step S102). Whether the voltage value of $2^1$ to $2^n \, \Omega$ applied to each of the resistors 3101 to 3111 deviates from the predetermined range is determined as described above (step S104). If the voltage value is within the predetermined range (appropriate), normal startup is performed (step S108). In contrast, if it is determined that the voltage value is anormal, the display unit displays an error (step S106).

The power supply controller 40 has a timer that measures time elapsed after disappearance of operation input, input and receiving of digital data, or the like to prevent a power supply switch 1a from being forgot to be turned off. After the elapse of a predetermined time (for example, ten minutes) after the disappearance of operation input, input and receiving of digital data, or the like, the power supply controller 40 outputs, to a power supply circuit, a power-off signal for turning off the power supply circuit.

When the powering off of the connected biological sensor 700 is detected, the power supply controller 40 preferably powers off the biological sensor 700. This is performed to prevent hindering of connection with a different biological sensor 700 after the connected biological sensor 700 is powered off, the hindering possibly occurring because the connected biological sensor 700 is stored in the conversion adapter 1. Note that instead of the powering off, a configuration in which information regarding the connected biological sensor 700 is discarded may be employed.

The connection unit 50 is connectable through wire to the external biological information monitor (bedside monitor) 800 and outputs, to the biological information monitor 800, the biological information based on the analog data converted by the converter 30 (for example, the combined series resistance value). A monaural phone plug with φ6.3 mm used for, for example, YSI400 series thermistor probes is preferably used for the connection unit 50. A terminal or a connector conforming to the same standard (of the same type) as that for the terminal or the connector of the applied wired biological information monitor 800 to which the connection unit 50 is thus used for the connection unit 50.

In the configuration as described above, the biological information based on the digital data wirelessly transmitted from the external biological sensor 700 is received, and the received biological information undergoes the data processing and is converted to the analog data conforming to the external biological information monitor (bedside monitor) 800. The analog data is output through wire by using the connection unit 50.

As described in detail above, this embodiment enables the biological information monitor (bedside monitor) 800 connected to the biological sensor 700 through wire to be used simply and at low cost as a wireless biological information monitor 800 (that is, the wireless capability to be added). In addition, the configuration in which the biological information from the biological sensor 700 is transmitted and received as the digital data is employed. Accordingly, as compared with a case where the biological information is transmitted and received as analog data, the quality of the radio communication information is kept high, and the wireless capability can be added easily to the existing biological information monitor 800.

According to this embodiment, the algorithmic process (such as the calibration process, the deep-body temperature estimation process, the balanced-state determination process, and the process for estimating a blood pressure level and·a blood-sugar level) of the biological information based on the digital data is performed on the conversion adapter 1 side. The processing load on the biological sensor 700 is thus reduced, and the power consumption is reduced. The life of the battery can be extended. In addition, for example, if the algorithm is changed, the configuration as above can reduce effort for firmware update involved with the change (if the number of conversion adapters 1 is lower than the number of biological sensors 700).

According to this embodiment, the conversion adapter includes the plurality of resistors 3101 to 3111 connected in series and the switching elements 3201 to 3212 that respectively turn on (enable) and off (disable) the plurality of resistors 3101 to 3111, and the resistance values of the plurality of resistors 3101 to 3111 are each set at $2^1$ to $2^n$ (n is a natural number). The calculation algorithm can thus be simplified, operating speed can be improved, and software development man-hours can be reduced. The mean value, the median, and the mode of the total resistance value of the switching elements 3201 to 3212 are obtained in advance as the correction values, implementation performed a plurality of times for higher accuracy can be omitted.

Further, according to this embodiment, the power is turned off after the elapse of the predetermined time after the disappearance of operation input and digital data input. Accordingly, even if the power is not turned off with the power supply switch 1a, a wasteful use of the battery can be prevented.

The embodiment of the present invention has heretofore been described; however, the present invention is not limited to the embodiment above, and various modifications may be made thereto. For example, in the embodiment above, the case where the temperature sensors and photoplethysmographic sensors are used as the biological sensor 700 has been described as an example, but a different biological sensor may also be used instead of the sensors. The type of the algorithmic process is not limited to that in the embodiment above. A configuration in which a process other than the calibration, the deep-body temperature estimation, the blood-pressure level estimation, and the blood-sugar level estimation may thus be employed. In addition, a configuration in which instead of or in addition to a blood pressure level and a blood sugar level, for example, an electrocardiogram, blood pressure, respiration, brain waves, a CO2 partial pressure curve, a pulse wave, a heart rate, a VPC count, a respiratory rate, a pulse rate, (highest, lowest, and mean) blood pressure, (highest, lowest, and mean) non-invasive blood pressure, body temperature, a temperature differential or blood temperature, ETCO2, tcPO2, tcPCO2, SpO2, or a ST level is acquired (measured) may also be employed.

(First Modification)

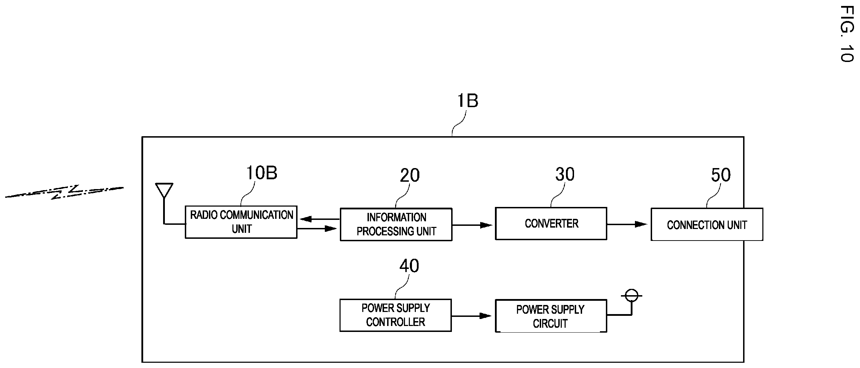

In addition to the configuration of the embodiment above, for example, as illustrated in Fig. 10, a configuration in which a radio communication unit 10B transmits information to an external medical apparatus by using radio communication may also be employed. More specifically, for example, a configuration enabling the biological information processed and generated by the information processing unit 20 to be transmitted to a different medical apparatus (such as an electric medical record system) having a radio receiving function via a radio communication unit 10B is preferably employed. According to this modification, the biological information processed and generated by a conversion adapter 1B can be transmitted to the different medical apparatus.

(Second Modification)

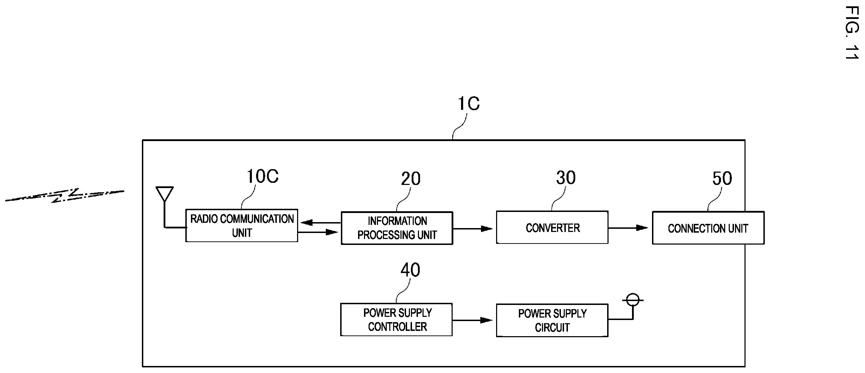

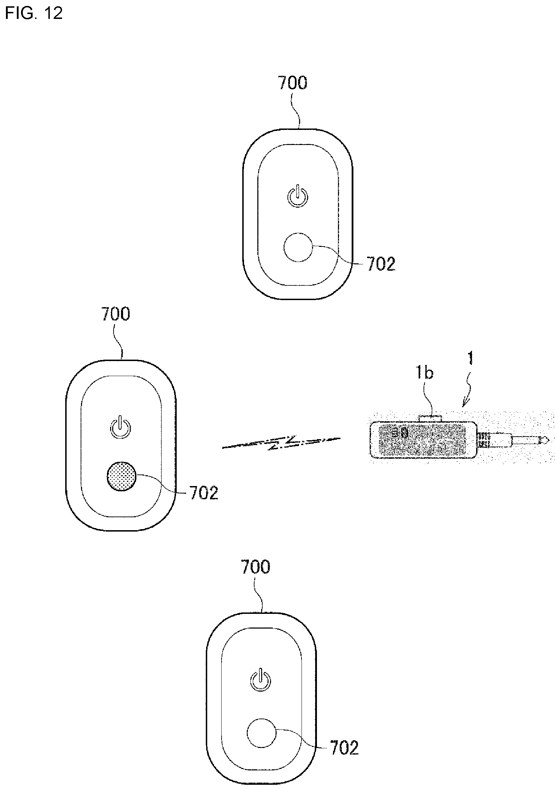

As illustrated in Fig. 11, a radio communication unit 10C may be configured to transmit, to the biological sensor 700, information (for example, verification information regarding connection with the biological sensor 700) by using radio communication. In this case, as illustrated in Fig. 12, for example, pressing the connection switch (push switch) 1b causes a connection verification command to be transmitted and a LED 702 of the biological sensor 700 connected (paired) wirelessly to light up or blink. Note that instead of the lighting or blinking of the LED 702, the biological sensor 700 may vibrate or sound a buzzer. According to this modification, if there are a plurality of biological sensors 700, the light emitting, the sound generation, or the like on the biological sensor 700 side in response to receiving the connection verification information enables a connection target to be verified.

(Third Modification)

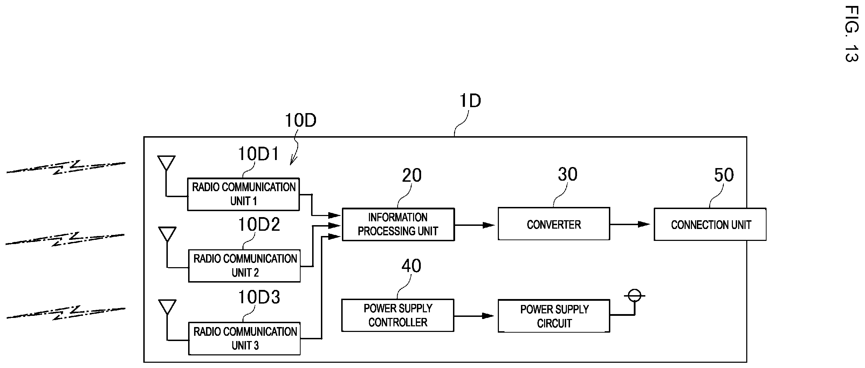

Further, as illustrated in Fig. 13, a radio communication unit 10D may be configured to support radio communication performed by a plurality of different radio communication methods. More specifically, for example, a configuration in which a plurality of radio communication units (radio communication drivers) (three radio communication units in the example in Fig. 13) 10D1 to 10D3 are provided may be employed. In this case, for the plurality of radio communication units (radio communication drivers) (three radio communication units in the example in Fig. 13) 10D1 to 10D3, radio communication methods (radio communication standards) such as Bluetooth (registered trademark) low energy (BLE), WiFi, long term evolution (LTE), and Sub-GHz (a 900 MHz band) and radio communication methods (radio communication standard) such as near field communication (NFC) (ISO/IEC 18092) and MIFARE (registered trademark) (ISO/IEC 14443) are preferably employed. This modification enables connection with the biological sensor 700 having various different communication drivers by using one conversion adapter 1D and can thus act as an intermediary for various pieces of sensor information in wide use.

Reference Signs List

| | |
|---|---|
| 1, 1B, 1C, 1D | conversion adapter |
| 10, 10B, 10C, 10D | radio communication unit |
| 10D1 to 10D3 | radio communication unit |
| 20 | information processing unit |
| 30 | converter |
| 3101 to 3111 | resistor |
| 3201 to 3212 | switching element |
| 40 | power supply controller |
| 50 | connection unit |
| 700 | biological sensor |
| 800 | biological information monitor |

**Claims**

1.  A conversion adapter (1, 1B, 1C, 1D) comprising:

    a radio communication unit (10, 10B, 10C, 10D) that is configured to receive biological information based on digital data transmitted from an external apparatus by using radio communication;
    an information processing unit (20) that is configured to perform data processing of the biological information based on the digital data received by the radio communication unit (10, 10B, 10C, 10D);
    a converter (30) that is configured to convert the biological information based on the digital data processed by the information processing unit (20) to biological information based on analog data; and
    a connection unit (50) that is connectable to an external biological-information processing apparatus and that is configured to output, to the biological-information processing apparatus, the biological information based on the analog data converted by the converter (30), **characterized in that** the information processing unit (20) is configured to perform an algorithmic process on the biological information based on the digital data, wherein the radio communication unit (10, 10B, 10C, 10D) is configured to receive biological information based on digital data transmitted from an external biological sensor (700) by using radio communication, and

wherein as the algorithmic process, the information processing unit (20) is configured to perform a calibration process in which variation in a characteristic of the biological sensor (700) is corrected.

2.   The conversion adapter (1, 1B, 1C, 1D) according to Claim 1,

wherein the converter (30) includes a plurality of resistors (3101-3111) connected in series and switching elements that are configured to turn on and off the respective plurality of resistors (3101-3111),

wherein resistance values of the plurality of resistors (3101-3111) are each set at $2^1$ to $2^n$ (n is a natural number), and

wherein the plurality of resistors (3101-3111) connected in series are each composed of one resistor or a plurality of resistors connected in series or in parallel.

3.   The conversion adapter (1, 1B, 1C, 1D) according to Claim 1,

wherein the radio communication unit is configured to receive a plurality of pieces of temperature information as the biological information, and

wherein based on the plurality of pieces of temperature information, the information processing unit is configured to estimate deep-body temperature as the algorithmic process.

4.   The conversion adapter (1, 1B, 1C, 1D) according to claim 1,

wherein the radio communication unit (10, 10B, 10C, 10D) is configured to receive a photoplethysmographic information as the biological information, and

wherein based on the photoplethysmographic information, the information processing unit is configured to estimate one or both of a blood pressure level and a blood sugar level as the algorithmic process.

5.   The conversion adapter (1, 1B, 1C, 1D) according to any one of Claims 1 to 4,
wherein the radio communication unit (10, 10B, 10C, 10D) is configured to transmit information to one or both of the biological sensor (700) and an external medical apparatus by using radio communication.

6.   The conversion adapter (1, 1B, 1C, 1D) according to Claim 5,
wherein the information is connection verification information regarding one or both of the biological sensor and the external medical apparatus.

7.   The conversion adapter according to any one of Claims 1 to 6,
wherein the radio communication unit (10, 10B, 10C, 10D) is configured to support radio communication performed by a plurality of different radio communication methods.

8.   The conversion adapter (1, 1B, 1C, 1D) according to any one of Claims 1 to 7, further comprising:
a power supply controller (40) that is configured to turn off the conversion adapter (1, 1B, 1C, 1D) after elapse of a predetermined time after disappearance of operation input and digital data input.

9.   The conversion adapter (1, 1B, 1C, 1D) according to any one of Claims 1 to 6,
wherein the information processing unit (20) is configured to perform a calibration process in which variation of the resistance values of the plurality of resistors (3101-3111) included in the converter (30) is corrected.

10.  The conversion adapter (1, 1B, 1C, 1D) according to any one of Claims 1 to 6,
wherein the information processing unit (20) is configured to perform a fault diagnosis process for the converter (30).

11.  The conversion adapter (1, 1B, 1C, 1D) according to any one of Claims 1 to 6,
wherein, from among a plurality of the biological sensors (700), the radio communication unit (10, 10B, 10C, 10D) is configured to select a biological sensor (700) having a highest radio wave intensity of a received radio wave as a biological sensor (700) with which connection is to be established.

**12.** The conversion adapter (1, 1B, 1C, 1D) according to Claim 11,
wherein in response to the radio wave intensity of the received radio wave varying by a predetermined width or dropping to a predetermined value or lower, the radio communication unit is configured to output information indicating that the radio wave intensity has varied by the predetermined width or that the radio wave intensity has dropped to the predetermined value or lower.

**13.** The conversion adapter (1, 1B, 1C, 1D) according to any one of Claims 1 to 6, further comprising:
a connection switch that is configured for establishing connection with the biological sensor, wherein the radio communication unit (10, 10B, 10C, 10D) is configured to establish the connection with the biological sensor (700) when the connection switch (1b) is pressed.

**14.** The conversion adapter (1, 1B, 1C, 1D) according to any one of Claims 1 to 6, further comprising:

a memory that stores the biological sensor (700) with which the connection is established, and
wherein in response to disconnection from the biological sensor (700) stored in the memory, the radio communication unit (10, 10B, 10C, 10D) is configured to retry the connection with the biological sensor (700).

**15.** The conversion adapter (1, 1B, 1C, 1D) according to Claim 8,
wherein in response to the connected biological sensor (700) being turned off, the power supply controller (40) is configured to turn off the conversion adapter (1, 1B, 1C, 1D).

**Patentansprüche**

**1.** Konvertierungsadapter (1, 1B, 1C, 1D), umfassend:

eine Funkkommunikationseinheit (10, 10B, 10C, 10D), die dafür ausgelegt ist, auf digitalen Daten basierende biologische Informationen zu empfangen, die von einer externen Vorrichtung mittels Funkkommunikation übertragen werden;
eine Informationsverarbeitungseinheit (20), die dafür ausgelegt ist, eine Datenverarbeitung der von der Funkkommunikationseinheit (10, 10B, 10C, 10D) empfangenen, auf digitalen Daten basierenden biologischen Informationen durchzuführen;
einen Konverter (30), der dafür ausgelegt ist, die von der Informationsverarbeitungseinheit (20) verarbeiteten, auf digitalen Daten basierenden biologischen Informationen in auf analogen Daten basierende biologische Informationen umzuwandeln; und
eine Verbindungseinheit (50), die mit einer externen Vorrichtung zur Verarbeitung biologischer Informationen verbindbar ist und dafür ausgelegt ist, der Vorrichtung zur Verarbeitung biologischer Informationen die vom Konverter (30) umgewandelten, auf analogen Daten basierenden biologischen Informationen zuzuführen, **dadurch gekennzeichnet, dass** die Informationsverarbeitungseinheit (20) dafür ausgelegt ist, einen algorithmischen Prozess auf den auf digitalen Daten basierenden biologischen Informationen auszuführen, wobei die Funkkommunikationseinheit (10, 10B, 10C, 10D) dafür ausgelegt ist, auf digitalen Daten basierende biologische Informationen zu empfangen, die von einem externen biologischen Sensor (700) mittels Funkkommunikation übertragen werden, und wobei die Informationsverarbeitungseinheit (20) als algorithmischen Prozess einen Kalibrierungsprozess ausführt, bei dem eine Variation einer Eigenschaft des biologischen Sensors (700) korrigiert wird.

**2.** Konvertierungsadapter (1, 1B, 1C, 1D) nach Anspruch 1,

wobei der Konverter (30) eine Vielzahl von in Reihe geschalteten Widerständen (3101-3111) sowie Schaltelemente aufweist, die dafür ausgelegt sind, die jeweilige Vielzahl von Widerständen (3101-3111) ein- und auszuschalten,
wobei die elektrischen Widerstandswerte der Vielzahl von Widerständen (3101-3111) jeweils auf $2^1$ bis $2^n$ eingestellt sind, wobei n eine natürliche ganze Zahl ist, und
wobei die in Reihe geschalteten Widerstände (3101-3111) jeweils aus einem Widerstand oder aus einer Vielzahl von in Reihe oder parallel geschalteten Widerständen bestehen.

**3.** Konvertierungsadapter (1, 1B, 1C, 1D) nach Anspruch 1,

wobei die Funkkommunikationseinheit dafür ausgelegt ist, eine Vielzahl von Temperaturinformationselementen als biologische Informationen zu empfangen, und
wobei die Informationsverarbeitungseinheit dafür ausgelegt ist, auf Grundlage der Vielzahl von Temperaturinformationselementen als algorithmischen Prozess eine Kerntemperatur des Körpers zu schätzen.

4. Konvertierungsadapter (1, 1B, 1C, 1D) nach Anspruch 1,

   wobei die Funkkommunikationseinheit (10, 10B, 10C, 10D) dafür ausgelegt ist, photoplethysmographische Informationen als biologische Informationen zu empfangen, und
   wobei die Informationsverarbeitungseinheit dafür ausgelegt ist, auf Grundlage der photoplethysmographischen Informationen als algorithmischen Prozess einen arteriellen Blutdruckwert und/oder einen Blutzuckerwert zu schätzen.

5. Konvertierungsadapter (1, 1B, 1C, 1D) nach einem der Ansprüche 1 bis 4,
   wobei die Funkkommunikationseinheit (10, 10B, 10C, 10D) dafür ausgelegt ist, Informationen mittels Funkkommunikation an den biologischen Sensor (700) und/oder an ein externes medizinisches Gerät zu übertragen.

6. Konvertierungsadapter (1, 1B, 1C, 1D) nach Anspruch 5,
   wobei die Informationen Verbindungsprüfinformationen bezüglich des biologischen Sensors und/oder des externen medizinischen Geräts sind.

7. Konvertierungsadapter (1, 1B, 1C, 1D) nach einem der Ansprüche 1 bis 6,
   wobei die Funkkommunikationseinheit (10, 10B, 10C, 10D) dafür ausgelegt ist, Funkkommunikation gemäß einer Vielzahl unterschiedlicher Funkkommunikationsverfahren zu unterstützen.

8. Konvertierungsadapter (1, 1B, 1C, 1D) nach einem der Ansprüche 1 bis 7, ferner umfassend:
   eine Stromversorgungssteuereinrichtung (40), die dafür ausgelegt ist, den Konvertierungsadapter (1, 1B, 1C, 1D) nach Ablauf einer vorgegebenen Zeit ohne Betriebs- und digitale Dateneingaben abzuschalten.

9. Konvertierungsadapter (1, 1B, 1C, 1D) nach einem der Ansprüche 1 bis 6,
   wobei die Informationsverarbeitungseinheit (20) dafür ausgelegt ist, einen Kalibrierungsprozess auszuführen, bei dem eine Variation der elektrischen Widerstandswerte der Vielzahl von im Konverter (30) enthaltenen Widerstände (3101-3111) korrigiert wird.

10. Konvertierungsadapter (1, 1B, 1C, 1D) nach einem der Ansprüche 1 bis 6,
    wobei die Informationsverarbeitungseinheit (20) dafür ausgelegt ist, einen Fehlerdiagnoseprozess für den Konverter (30) auszuführen.

11. Konvertierungsadapter (1, 1B, 1C, 1D) nach einem der Ansprüche 1 bis 6,
    wobei die Funkkommunikationseinheit (10, 10B, 10C, 10D) aus einer Vielzahl biologischer Sensoren (700) den biologischen Sensor (700) auswählt, der die höchste Feldstärke einer empfangenen Funkwelle aufweist, als den biologischen Sensor (700), mit dem eine Verbindung herzustellen ist.

12. Konvertierungsadapter (1, 1B, 1C, 1D) nach Anspruch 11,
    wobei die Funkkommunikationseinheit dafür ausgelegt ist, Informationen auszugeben, die anzeigen, dass die Feldstärke der empfangenen Funkwelle sich um eine vorgegebene Breite verändert hat oder auf einen vorgegebenen Wert oder darunter gefallen ist, als Reaktion darauf, dass die Feldstärke der empfangenen Funkwelle sich um die vorgegebene Breite verändert oder auf den vorgegebenen Wert oder darunter gefallen ist.

13. Konvertierungsadapter (1, 1B, 1C, 1D) nach einem der Ansprüche 1 bis 6, ferner umfassend:

    einen Verbindungsschalter, der dafür ausgelegt ist, eine Verbindung mit dem biologischen Sensor herzustellen, wobei die Funkkommunikationseinheit (10, 10B, 10C, 10D) dafür ausgelegt ist, die Verbindung mit dem biologischen Sensor (700) herzustellen, wenn der Verbindungsschalter (1b) betätigt wird.

14. Konvertierungsadapter (1, 1B, 1C, 1D) nach einem der Ansprüche 1 bis 6, ferner umfassend:

    einen Speicher zum Speichern des biologischen Sensors (700), mit dem die Verbindung hergestellt ist,

wobei die Funkkommunikationseinheit (10, 10B, 10C, 10D) dafür ausgelegt ist, als Reaktion auf eine Trennung von dem im Speicher gespeicherten biologischen Sensor (700) erneut zu versuchen, die Verbindung mit dem biologischen Sensor (700) herzustellen.

15. Konvertierungsadapter (1, 1B, 1C, 1D) nach Anspruch 8,
    wobei die Stromversorgungssteuereinrichtung (40) dafür ausgelegt ist, den Konvertierungsadapter (1, 1B, 1C, 1D) abzuschalten, wenn der verbundene biologische Sensor (700) abgeschaltet wird.

**Revendications**

1. Adaptateur de conversion (1, 1B, 1C, 1D) comprenant :

   une unité de radiocommunication (10, 10B, 10C, 10D) conçue pour recevoir des informations biologiques fondées sur des données numériques transmises à partir d'un dispositif externe à l'aide d'une communication radio ;
   une unité (20) de traitement d'informations conçue pour effectuer un traitement de données des informations biologiques fondées sur des données numériques reçues par l'unité de radiocommunication (10, 10B, 10C, 10D) ;
   un convertisseur (30) conçu pour convertir les informations biologiques fondées sur des données numériques, traitées par l'unité (20) de traitement d'informations, en informations biologiques fondées sur des données analogiques ; et
   une unité de connexion (50) pouvant être connectée à un dispositif externe de traitement d'informations biologiques, et étant conçue pour délivrer, au dispositif de traitement d'informations biologiques, les informations biologiques fondées sur les données analogiques converties par le convertisseur (30),
   **caractérisé en ce que** l'unité (20) de traitement d'informations est conçue pour effectuer un processus algorithmique sur les informations biologiques fondées sur les données numériques,
   dans lequel l'unité de radiocommunication (10, 10B, 10C, 10D) est conçue pour recevoir des informations biologiques fondées sur des données numériques transmises à partir d'un capteur biologique externe (700) à l'aide d'une communication radio, et
   dans lequel, en tant que processus algorithmique, l'unité (20) de traitement d'informations est conçue pour effectuer un processus d'étalonnage dans lequel une variation d'une caractéristique du capteur biologique (700) est corrigée.

2. L'adaptateur de conversion (1, 1B, 1C, 1D) selon la Revendication 1,

   dans lequel le convertisseur (30) comporte une pluralité de résistances (3101-3111) connectées en série et des éléments de commutation qui sont conçus pour mettre sous tension et hors tension la pluralité respective de résistances (3101-3111),
   dans lequel les valeurs de résistance électrique de la pluralité de résistances (3101-3111) sont réglées chacune sur $2^1$ à $2^n$ (n étant un nombre entier naturel), et
   dans lequel la pluralité de résistances (3101-3111) connectées en série sont composées chacune d'une résistance ou d'une pluralité de résistances connectés en série ou en parallèle.

3. L'adaptateur de conversion (1, 1B, 1C, 1D) selon la Revendication 1,

   dans lequel l'unité de radiocommunication est conçue pour recevoir une pluralité d'éléments d'informations de température en tant qu'informations biologiques, et
   dans lequel, sur la base de la pluralité d'éléments d'informations de température, l'unité de traitement d'informations est conçue pour estimer une température corporelle profonde en tant que processus algorithmique.

4. L'adaptateur de conversion (1, 1B, 1C, 1D) selon la Revendication 1,

   dans lequel l'unité de radiocommunication (10, 10B, 10C, 10D) est conçue pour recevoir des informations photopléthysmographiques en tant qu'informations biologiques, et
   dans lequel, sur la base des informations photopléthysmographiques, l'unité de traitement d'informations est conçue pour estimer l'un et/ou l'autre parmi un niveau de tension artérielle et un niveau de sucre dans le sang en tant que processus algorithmique.

**5.** L'adaptateur de conversion (1, 1B, 1C, 1D) selon l'une quelconque des Revendications 1 à 4,
dans lequel l'unité de radiocommunication (10, 10B, 10C, 10D) est conçue pour transmettre des informations à l'un et/ou l'autre parmi le capteur biologique (700) et un dispositif médical externe, à l'aide d'une communication radio.

**6.** L'adaptateur de conversion (1, 1B, 1C, 1D) selon la Revendication 5,
dans lequel les informations sont des informations de vérification de connexion concernant l'un et/ou l'autre parmi le capteur biologique et le dispositif médical externe.

**7.** L'adaptateur de conversion (1, 1B, 1C, 1D) selon l'une quelconque des Revendications 1 à 6,
dans lequel l'unité de radiocommunication (10, 10B, 10C, 10D) est conçue pour soutenir des communications radio effectuées par une pluralité de procédés de radiocommunication différents.

**8.** L'adaptateur de conversion (1, 1B, 1C, 1D) selon l'une quelconque des Revendications 1 à 7, comprenant en outre :
un dispositif de commande d'alimentation électrique (40) conçu pour mettre hors tension l'adaptateur de conversion (1, 1B, 1C, 1D) après écoulement d'un temps prédéfini après l'absence d'entrées de fonctionnement et d'entrées de données numériques.

**9.** L'adaptateur de conversion (1, 1B, 1C, 1D) selon l'une quelconque des Revendications 1 à 6,
dans lequel l'unité (20) de traitement d'informations est conçue pour effectuer un processus d'étalonnage dans lequel une variation des valeurs de résistance électrique de la pluralité de résistances (3101-3111), comprises dans le convertisseur (30), est corrigée.

**10.** L'adaptateur de conversion (1, 1B, 1C, 1D) selon l'une quelconque des Revendications 1 à 6,
dans lequel l'unité (20) de traitement d'informations est conçue pour effectuer un processus de diagnostic d'anomalies destiné au convertisseur (30).

**11.** L'adaptateur de conversion (1, 1B, 1C, 1D) selon l'une quelconque des Revendications 1 à 6,
dans lequel, parmi une pluralité des capteurs biologiques (700), l'unité de radiocommunication (10, 10B, 10C, 10D) est conçue pour choisir un capteur biologique (700), présentant la plus haute intensité d'onde radioélectrique d'une onde radioélectrique reçue, en tant que capteur biologique (700) avec lequel une connexion doit être établie.

**12.** L'adaptateur de conversion (1, 1B, 1C, 1D) selon la Revendication 11,
dans lequel, en réponse au fait que l'intensité d'onde radioélectrique de l'onde radioélectrique reçue varie d'une largeur prédéfinie ou chute à une valeur égale ou inférieure à une valeur prédéfinie, l'unité de radiocommunication est conçue pour délivrer des informations indiquant que l'intensité d'onde radioélectrique a varié de la largeur prédéfinie ou que l'intensité d'onde radioélectrique a chuté à la valeur égale ou inférieure à la valeur prédéfinie.

**13.** L'adaptateur de conversion (1, 1B, 1C, 1D) selon l'une quelconque des Revendications 1 à 6, comprenant en outre :
un commutateur de connexion conçu pour établir une connexion avec le capteur biologique, et dans lequel l'unité de radiocommunication (10, 10B, 10C, 10D) est conçue pour établir la connexion avec le capteur biologique (700) lorsque le commutateur de connexion (1b) est pressé.

**14.** L'adaptateur de conversion (1, 1B, 1C, 1D) selon l'une quelconque des Revendications 1 à 6, comprenant en outre :

une mémoire servant à stocker le capteur biologique (700) avec lequel la connexion est établie, et
dans lequel, en réponse à une déconnexion à partir du capteur biologique (700) stocké dans la mémoire, l'unité de radiocommunication (10, 10B, 10C, 10D) est conçue pour tenter à nouveau la connexion avec le capteur biologique (700).

**15.** L'adaptateur de conversion (1, 1B, 1C, 1D) selon la Revendication 8,
dans lequel, en réponse à une mise hors tension du capteur biologique connecté (700), le dispositif de commande d'alimentation électrique (40) est conçu pour mettre hors tension l'adaptateur de conversion (1, 1B, 1C, 1D).

FIG. 1

FIG. 2

FIG. 3

EP 4 129 159 B1

FIG. 3

FIG. 4

Temperature output characteristic after a digital-calibration of each thermistor

Temperature output characteristic before calibration

FIG. 5

701

701a ROOM
TEMPERATURE Ta                    701b

712 —

715 —

711 —

T2

T1

T4

714

716

713

T3

IpA                              IpB        Body

DEEP-BODY TEMPERATURE Tb

FIG. 6

PHOTOPLETHYSMOGRAPHIC WAVEFORM

FIG. 7

FIG. 8

RESISTANCE RANGE : 0Ω~4094Ω    STEP : 2Ω

FIG. 9

TOTAL SWITCH RESISTANCE
VALUE DISTRIBUTION

FIG. 10

FIG. 11

FIG. 12

FIG. 13

FIG. 14

```
                    ┌─────────────────────┐
                    │        START        │
                    └─────────────────────┘
                               │
                               ▼
                    ┌─────────────────────┐
                    │ TURN ON AND START UP│────  S100
                    │  CONVERSION ADAPTER │
                    └─────────────────────┘
                               │
                               ▼
                    ┌─────────────────────┐
                    │  PERFORM SELF-CHECK │────  S102
                    └─────────────────────┘
                               │
                               ▼
                         ╱───────────╲              Yes
                       ╱  IS THERE FAULT? ╲─────────────────────┐
                       ╲               ╱  S104                  │
                         ╲───────────╱                         │
                               │ No                            ▼
                               ▼                    ┌─────────────────────┐
                    ┌─────────────────────┐         │    DISPLAY ERROR    │──── S106
                    │  START UP CONVERSION│──── S108 └─────────────────────┘
                    │   ADAPTER NORMALLY  │                    │
                    └─────────────────────┘                    │
                               │◄───────────────────────────────┘
                               ▼
                    ┌─────────────────────┐
                    │        END          │
                    └─────────────────────┘
```

**REFERENCES CITED IN THE DESCRIPTION**

**Patent documents cited in the description**

- JP 2007215582 A
- JP 2018527996 W
- US 2005119533 A1
- US 2005119533 A